# EUROPEAN PATENT SPECIFICATION

(11) **EP 0 961 604 B1**
(45) Date of publication and mention of the grant of the patent: **22.05.2002**
(21) Application number: 97951354.6
(22) Date of filing: 12.12.1997
(51) Int. Cl.: A61F 13/15

(54) **SURFACE MATERIAL FOR ABSORBENT ARTICLES, ABSORBENT ARTICLES THAT INCLUDE THE SURFACE MATERIAL, AND THE USE OF THE MATERIAL IN ABSORBENT ARTICLES**
OBERFLÄCHENMATERIAL FÜR ABSORBIERENDE ARTIKEL, ABSORBIERENDE ARTIKEL MIT DEM OBERFLÄCHENMATERIAL UND DIE BENUTZUNG DIESES MATERIALS IN ABSORBIERENDEN ARTIKEL
MATERIAU DE SURFACE POUR ARTICLES ABSORBANTS, ARTICLES ABSORBANTS CONTENANT CE MATERIAU DE SURFACE ET UTILISATION DUDIT MATERIAU DANS DES ARTICLES ABSORBANTS

(30) Priority: 30.12.1996 SE 9604833
(43) Date of publication of application: 08.12.1999
(73) Proprietor: SCA Hygiene Products AB, 405 03 Göteborg (SE)
(72) Inventor: MAGNUSSON, IngBritt, S-435 37 Mölnlycke (SE); OLOFSSON, Ulla, S-430 94 Bohus-Björkö (SE)
(74) Representative: Larsson, Karin
(86) International application number: SE9702074
(87) International publication number: WO9829071

(56) References cited:
- EP-A- 0 729 735
- WO-A-93/15249
- WO-A-95/17867
- FILE WPI, Derwent Accession No. 91-002963, KURARAY CO LTD, "Surface Material for Hygienic Material - Contains Spirally Crimped Eccentric Core-Sheath Polyester Composited Fibre and Binder Fibre Which are Heat Fused"; & JP,A,02 279 154 (15-11-90) DW9101.

## Description

### BACKGROUND OF THE INVENTION

The present invention relates to a surface material for absorbent articles, such as diapers, sanitary napkins, incontinence guards, panty liners and like articles, and to absorbent articles that include said surface material, and also to the use of this material as a combined outer sheet and acquisition/transportation sheet in an absorbent article.

Such a surface material is known from the document EP-A-0 696 655.

An absorbent article is typically comprised of several layers or sheets of material, e.g. a liquid-impermeable backing sheet, an absorbent layer or sheet disposed on the backing sheet, followed by an acquisition layer/transport layer and finally a surface sheet or top sheet which is intended to lie proximal to the wearer in use. The acquisition/transport layer has an open and airy structure and functions to quickly take up a given volume of liquid and to conduct this liquid quickly to the absorbent layer. The acquisition/transportation sheet may be comprised of a high loft material, which may be produced by, e.g., through-air bonding or needling synthetic fibres, such as polyester, polypropylene fibres or mixtures thereof. Because this layer, or sheet, obtains a coarse and scratchy surface, it is undesirable for the layer to lie in immediate contact with the wearer's skin. It is therefore necessary to place on top of the acquisition sheet a surface sheet that is softer and more comfortable to the touch The surface sheet may be comprised of nonwoven material, for instance.

Different layered materials are known to the art, for instance from SE-B-470 064. This publication teaches an absorbent fibre structure that includes a sheet comprised of bicomponent fibres and superabsorbent material. The bicomponent fibres consist of heat-shrunk, spiralled, elastic thermoplastic bicomponent fibres, the fibre components having mutually different shrinkage properties and being positioned in mutually side-by-side relationship. The superabsorbent material is in particle form and is retained in the fibre structure chiefly by being locked mechanically in the turns of the spiralled bicomponent fibres. This absorbent fibre structure is used in the absorption sheet of absorbent products and the main purpose of the bicomponent fibres is to retain the superabsorbent material so as to provide an absorbent sheet of good absorbency.

US-A-4,551,378 teaches a nonwoven stretch fabric. The fabric is produced from bicomponent fibres that are mutually bonded by fusing the fibres together at contact points while shlinking the fibres thermally at the same time. Because the fibre components have mutually different shrinkage properties, they form a three-dimensioiial spiral when heated. The fabric can be used as thermal insulation in sports clothing and in outdoor working garments.

EP-A1-0 306 262 and GB-A-2 214 201 teach absorbent structures in diapers and sanitary napkins, for instance. These structures have included therein curled fibres with the intention of imparting elasticity to the structure and also of enhancing pleating resistance during use of the absorbent product.

EP-A-0729735 discloses an absorbent composite, wherein the composite comprises at least one liquid absorbing layer and at least one material layer which is dry formed on the absorbing layer, from fibres crimping by means of heat.

File WP1, Derwent accession No. 91-002963, Kuraray Co Ltd, abstract of JP-A-2279154, 901115 describes a surface material comprising polyester composite fibre in the form of spiral crimping, a heat-fusing binder fibre and preferably one or a mixture of natural fibres, regenerated fibres, polyesters, polyamides, polyacrylonitriles, polyolefins, polyvinyl alcohols and polyvinyl chlorides. In a new preparation of the surface material, wet paper is paper-made from an aqueous dspersed solution of a fibre mixture, the wet paper is then heat-treated.

Some of the problems encountered with present-day absorbent articles are that the acquisition/transport sheets are expensive and, because of their coarse and scratchy surface, feel unpleasant when in contact with the skin. This necessitates the use of an additional, softer surface sheet against the body, which makes the article more expensive and more difficult to produce, since production is laboured with an additional sheet.

The object of the present invention is to solve these problems.

### SUMMARY OF THE INVENTION

The invention relates partly to surface material for absorbent articles, such as diapers, sanitary napkins, incontinence guards, panty liners and like articles, the material including heat-shrunk, spiralled, elastic thermoplastic multicomponent fibres, preferably bicomponent fibres. The spiralled fibres give the material an open and airy structure which enables liquid to be quickly taken into and transported through the surface sheet and to the underlying absorbent layer. The surface material also has a soft and smooth surface.

The inventive absorbent article includes a bottom liquid-impermeable sheet, an absorbent sheet and a top liquid-permeable surface sheet comprised of said surface material and intended to lie proximal to the wearer in use.

The invention also relates to the use of the above-described material as a combined surface and acquisition/transportation sheet in absorbent articles. Because the surface material has an open and airy structure, it will function as an acquisition/transportation sheet. The sheet is also soft and smooth, and can therefore function as a surface sheet at the same time.

The material has a weight per unit area of about 40-100 g/m², preferably about 50-90 g/m².

The invention will now be described in more detail with reference to the accompanying drawings, in which
Figure 1 is a schematic view of a spiralized bicomponent fibre;
Figures 2a, 2b and 2c are respective enlarged sectional views of three examples of mutually side-by-side fibres, taken on the line II-II in Figure 1;
Figure 3 is a schematic view of spiralized bicomponent fibres that have been fused together at certain points;
Figure 4 is sectional view of an incontinence guard;
Figure 5 illustrates an incontinence guard from above;
Figure 6 is a photograph of one embodiment of the inventive material, taken by a scanning electron microscope at an enlargement of 100 times; and
Figure 7 is a photograph showing a cross-section through the fibres of the material shown in Figure 6, and enlarged 1000 times.

### DETAILED DESCRIPTION OF THE INVENTION

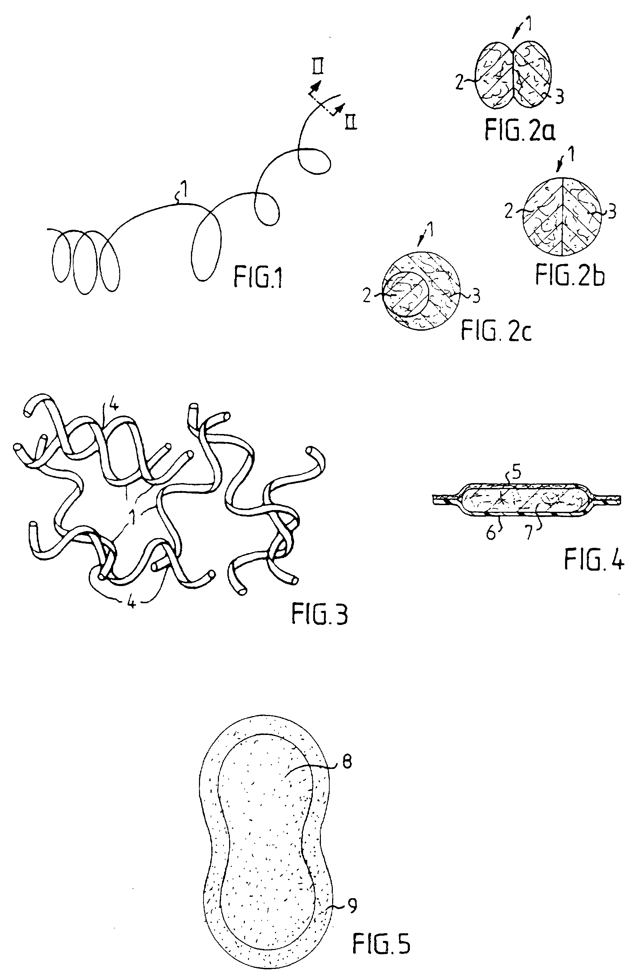

The invention relates to a surface material according to claim 1. A heat-shrunk, spiralized elastic bicomponent fibre is shown in Figure 1.

The invention is described below with respect to a bicomponent fibre.

Different types of mutually juxtaposed bicomponent fibres can be used. Figure 2a is a cross-sectional view of the fibre 1 in which both components 2, 3 lie side-by-side and have an elliptical shape and are bonded together along parts of the surfaces of respective fibres. Figure 2b illustrates another type of fibre in which the components 2, 3 are mutually juxtaposed similar to the Figure 2a illustiation. In this latter case, however, the bicomponent fibre 1 has a circular cross-section with an interconnecting line between the two components 2, 3 through the bicomponent fibre section. Figure 2c shows a third type of fibre which, in this case, is an eccentric skin-core fibre. The first component 2 of the fibre 1 has the form of a core that is placed eccentrically in the surrounding skin of the second component 3. The core containing the first component 2 is placed in the second component 3 such that the second component will leave bare that part of the surface of the first component 2 which lies at the periphery or bordeer of the bicomponent fibre. The first component 2 will thus have a freely-lying outer part.

Although bicomponent fibres are usually round, fibres that have other cross-sectional shapes are available, for instance fibres having elliptical, trilobal or rectangular cross-sectional shapes.

It is essential that the fibre components chosen will have mutually different shrinkage properties. When heat is applied to a bicomponent fibre of this nature. each of the two components will shrink to a different extent to the other, such that one side of the fibre will be shorter than the other side and therewith cause the fibre to twist and contract into the spiral shape shown in Figure 1. A bicomponent fibre suitable for this purpose is marketed by Clusso Corporation. Osaka, Japan, under reference No. EP-HS P2. This fibre is a 1.7 dtex thick shrink and melt fibre consisting of two different polypropylenes that have a high and a low melting point. respectively. This fibre is of the side-by-side type shown in Figure 2c.

Surface material comprised of these bicomponent fibres is produced by, e.g., carding a gauze fabric from juxtaposed fibres that are then oven-bonded. There are used staple fibres having a length of 30-60 mm, preferably 40-50 mm. The fibre components will shrink to different extents, causing the bicomponent fibre to twist and contract to a spiral shape. The heat-treated and bonded sheet will be thicker than the carded gauze that enters the oven, since the bicomponent fibres have spiralled and imparted an arier structure to the sheet. Because the bicomponent fibres partially melt in the heat-treatment process, the fibres also act as a binder in the actual fibre structure. The spirals will thus melt and fuse together at discrete points of the spirals, as shown in Figure 3. The bicomponent fibres 1 are mutually bonded at points 4.

The surface material is comprised suitably of hydrophobic fibres. The spiralled fibres give an open structure to the surface material. This means that the material will not absorb liquid, but instead provide rapid acquisition and transportation of liquid into the product. The surface material thus functions as an acquisition/transportation sheet in the absorbent article. Because the surface sheet allows liquid to pass quickly therethrough, the surface of the sheet will be felt to be dry against the wearer's skin in use. Movement of liquid on the surface of the product is prevented.

The inventive surface material is soft and smooth, as opposed to current acquisition/transportation sheets. The material is produced from fibres having a thickness of about 1.5-3.3 dtex, preferably 1.7-2.5 dtex. These fibres are relatively fine in comparison with the fibres used in conventional acquisition/transportation sheets, these typical fibres having a thickness of about 6 dtex mixed with binding fibres having a thickness of about 4 dtex. The coarser the fibres, the harder and more scratchy the material. This conventional sheet is too hard and too scratchy to be used in immediate contact with the wearer's skin. The aforesaid fibre thicknesses are necessary, however, in order to impart to this conventional material a structure that is sufficiently airy or open to function as an acquisition/transportation sheet. The bicomponent fibres that are used in the inventive material and have a thickness of about 1.5-3.3 dtex make the material smoother and softer than the conventional material and enable the inventive material to be placed proximal to the wearer's skin and in contact therewith. As will be understood, the sheet functions both as an acquisition/transportation sheet and as a surface sheet and therefore obviates the need of an additional surface sheet per se.

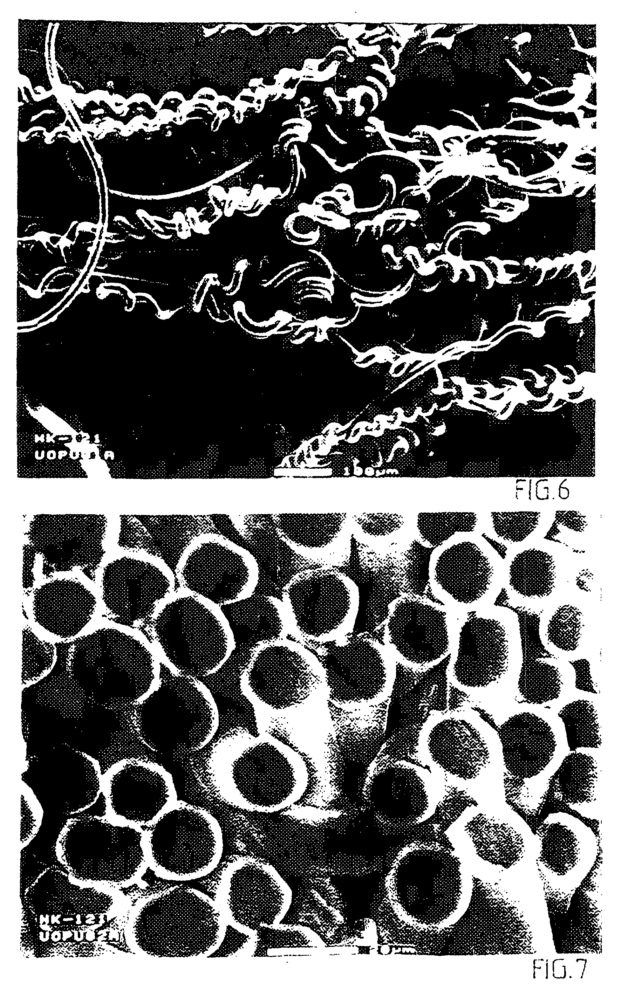

Figure 6 is a photograph which illustrates one embodiment of an inventive material produced from bicomponent fibres marketed by Chisso Corporation, Osaka, Japan, under reference No. EP-HS P2 and having a thickness of 1.7 dtex. The bicomponent fibre is a shrink and melt fibre comprised of two mutually different polypropylenes.

The fibre is enlarged 100 times in the photograph. It will be seen from the photograph that the fibres have spiralled as a result of the heat treatment.

Figure 7 is a photograph of the same material as that shown in Figure 6, although enlarged 1000 times. The photograph shows a cross-section of the fibres, from which it will be seen that the fibres are in mutually juxtaposed relationship in accordance with Figure 2c. The component 2 is seen as a core with component 3 disposed around the core. The component 3 has melted in the heat-treatment process and flowed so as to fully cover the core comprised of component 2.

It is essential that each of the two components 2, 3 in the shrink fibres 1 will shrink to a different extent to the other when heated. This shrinkage disparity causes the shrunk fibres to spiral. The shrunk bicomponent fibres 1 are highly extensible and resilient, therewith providing a measure of freedom of movement in the sheet The material can therewith easily be shaped in the finished product and will follow the contours of the wearer's body in a comfortable fashion.

In one preferred embodiment, the components 2, 3 of the bicomponent fibres 1 are comprised respectively of different types of polypropylene that have mutually different melting points and shrinkage properties.

In another embodiment, the components 2, 3 of the bicomponent fibres 1 are polypropylene and polyester respectively.

In yet another embodiment. the surface material also includes thermoplastic binding fibres of polypropylene or polyethylene, for instance.

The various polymers may also include other homopolymers or copolymers. Examples of polymers that can be used in this regard are polyolefins. polyamides, copolyamides. polyesters, copolyesters, polyacrylics and the like. The polymers may also contain different conventional additives in accordance with known practice, such as softeners, for instance. The feature of the invention is that the two components 2, 3 of the bicomponent fibre 1 have mutually different shrinkage properties.

An embodiment which includes a mixture of different bicomponent fibres is also conceivable.

When the surface material contains solely bicomponent fibres, it will have a very open and airy structure. In the case of the embodiment in which the material also includes thermoplastic binding fibres, these fibres will not shrink and spiral in the same way as the bicomponent fibres. Thus, a sheet that also includes binding fibres will not be as open or airy, nor yet as thick, as a sheet that includes solely bicomponent fibres. Binding fibres can be used to control or regulate the thickness and airiness of a sheet. When the material includes binding fibres, the binding fibres are carded together with the bicomponent fibres to form a gauze, which is then oven bonded.

The invention also relates to an absorbent article according to claim 7. The surface sheet is comprised of the inventive sheet material as described above.

Figure 4 is a cross-sectional view of an incontinence guard that includes an inventive surface sheet 5. The incontinence guard also includes a liquid-impermeable backing sheet 6, an absorbent sheet 7 next to the backing sheet, and, aboe the absorbent sheet 7, a liquid-permeable top sheet or surface sheet 5 that is intended to lie proximal to the wearer in use. The top surface sheet 5 is comprised of said heat-shrunk, spiralled, elastic thermoplastic bicomponent fibres 1, which are also hydrophobic. The surface sheet has an open and airy structure and also has a smooth and soft surface. The top surface sheet also functions as an acquisition/transportation sheet and allows liquid to quickly pass therethrough and be transported to the absorbent sheet 7. The smoothness and softness of the surface sheet enables it to lie comfortably against the wearer's skin. Thus, the surface sheet 5 is intended to face towards the wearer and lie in contact with the wearer's skin when the absorbent article is worn, and forms both a surface sheet and an acquisition/transportation sheet.

Figure 5 illustrates another embodiment of an incontinence guard from above. The incontinence guard comprises a liquid-impermeable bottom sheet 6, an absorbent sheet or layer 7, and a liquid-permeable top sheet or surface sheet 5 which lies proximal to the wearer in use. The surface sheet 5 is comprised of two different materials, firstly the inventive surface material 8 and secondly a nonwoven material 9. The inventive surface material 8 is placed in the wetting zone and a hydrophobic, nonwoven material 9 is placed at the edge of the incontinence guard around the inventive surface material 8. The nonwoven material 9 is essentially liquid impervious and is intended to prevent the reflux of liquid from the absorbent layer, so that no liquid can penetrate out towards the sides of the article. The nonwoven material can be welded together with the inventive surface material, for instance.

The invention also relates to the use of the aforesaid material according to claim 18.

As before mentioned, the material that includes the heat-shrunk, spiralled, elastic thermoplastic bicomponent fibres 1 will have a very airy and very open structure. Because the fibres are hydrophobic, the material will not absorb liquid but will, instead, allow liquid to pass quickly therethrough and be transported to the absorbent sheet 7. The inventive material has a smooth and soft surface. This enables the material to be used in the sheet that lies proximal to the wearer in use, without chafing the wearer or feeling uncomfortable to the touch. The material can then be used both as an acquisition/transportation sheet and as a surface sheet. This is advantageous in comparison with materials that are used at present to this end. Present-day acquisition/transportation sheets have scratchy and coarse surfaces, which render such sheets unsuitable for use in direct contact with the wearer's skin This means that an additional surface sheet is required in the case of conventional articles. The inventive material obviates the requirement of such an addinonal surface sheet.

It will be understood that the invention is not restricted to the described and illustrated embodiments thereof and that further modifications are conceivable within the scope of the following Claims.

## Claims

1. Surface material for absorbent articles such as diapers, sanitary napkins, incontinence guards, panty liners and like articles, wherein the surface material (5) includes heat-shrunk, spiralled, elastic thermoplastic bicomponent fibres (1), the components (2, 3) in said fibres having mutually different shrinkage properties and lying in side-by-side relationship and the components (2, 3) being hydrophobic, **characterized in that** the fibres (1) have a length of 30-60mm, and the components (2, 3) in the bicomponent fibres (1) each consists of a different type of polypropylene or the components (2, 3) of the bicomponent fibres (1) are polypropylene and polyester, respectively.

2. Surface material according to claim 1, **characterized in that** the fibres have a length of 40-50 mm.

3. Surface material according to Claim 1 or 2, **characterized in that** said material includes a mixture of bicomponent fibres.

4. Surface material according to any one of the preceding Claims, **characterized in that** said material also includes thermoplastic binding fibres of polypropylene or polyethylene, for instance.

5. Surface material according to any one of the preceding Claims, **characterized in that** the bicomponent fibres have a thickness of about 1.5-3.3 dtex or 1,7-2.5 dtex.

6. Surface material according to any one of the preceding Claims, **characterized in that** said material has a weight per unit area of about 40-100 g/m² or 50-90 g/m².

7. An absorbent article, such as a diaper, sanitary napkin, incontinence guard, panty liner or like article, comprising a liquid-impermeable backing sheet (6), an absorbent sheet or layer (7), and a liquid-permeable top surface sheet (5) which is intended to lie proximal to the wearer in use, wherein the surface sheet material includes heat-shrunk, spiralled, elastic thermoplastic bicomponent fibres (1), the components (2, 3) in said fibres having mutually different shrinkage properties and lying in a side-by-side relationship and the components (2, 3) being hydrophobic, **characterized in that** the fibres (1) have a length of 30-60 mm and that the components (2, 3) in the bicomponent fibres (1) consist of mutually different types of polypropylene or the components (2, 3) in the bicomponent fibres (1) are polypropylene and polyester, respectively.

8. An absorbent article according to claim 7, **characterized in that** the fibres have a length of 40-50 mm.

9. An absorbent article according to Claim 7 or 8, **characterized in that** the surface sheet (5) forms a combined surface sheet and acquisition/transportation sheet.

10. An absorbent article according to Claim 7 or 8, **characterized in that** the surface sheet material includes a mixture of bicomponent fibres.

11. An absorbent article according to any one of Claims 7-10, **characterized in that** the surface material (5) also includes thermoplastic binding fibres of polypropylene or polyethylene, for instance.

12. An absorbent article according to any one of Claims 7-11, **characterized in that** the bicomponent fibres have a thickness of about 1.5-3.3 dtex or 1.7-2.5 dtex.

13. An absorbent article according to any one of Claims 7-12, **characterized in that** the surface sheet has a weight per unit area of about 40-100 g/m² or 50-90 g/m².

14. The use of material that includes heat-shrunk, spiralized, elastic thermoplastic bicomponent fibres (1), the components (2, 3) of said fibres being hydrophobic and having mutually different shrinkage properties and lying in a side-by-side relationship, as a combined surface sheet and acquisition/transportation sheet (5) in absorbent articles, such as diapers, sanitary napkins, incontinence guards, panty liners and like articles that include beneath the combined surface sheet and acquisition/transportation sheet (5) an absorbent sheet (7) and a liquid-impermeable backing sheet (6), **characterized in that** the fibres have a length of 30-60 mm, and the components (2, 3) of the bicomponent fibres (1) are mutually different polypropylenes or components (2, 3) of the bicomponent fibres (1) are polypropylene and polyester, respectively.

15. The use of a material according to Claim 14, wherein the fibres (1) have a length of 40-50 mm.

16. The use of a material according to Claim 14 or 15, wherein the material includes a mixture of bicomponent fibres.

17. The use of a material according to any one of Claims 14-16, wherein the material further includes thermoplastic binding fibres of polypropylene or polyethylene, for instance.

18. The use of a material according to any one of Claims 14-17, wherein the bicomponent fibres have a thickness of about 1.5-3.3 dtex or 1.7-2.5 dtex.

19. The use of a material according to any one of Claims 14-18, wherein the material has a weight per unit area of about 40-100 g/m² or 50-90 g/m².

## Patentansprüche

1. Oberflächenmaterial für Absorptionsartikel, wie z.B. Windeln, Hygienebinden, Inkontinenzschutzeinrichtungen, Slipeinlagen und ähnliche Artikel, wobei das Oberflächenmaterial (5) wärmegeschrumpfte, spiralisierte, elastische, thermoplastische Zweikomponentenfasern (1) aufweist, wobei die Komponenten (2, 3) in den Fasern zueinander unterschiedliche Schrumpfungseigenschaften aufweisen und in einer Seite-an-Seite-Beziehung liegen, und wobei die Komponenten (2, 3) wasserabweisend sind,
**dadurch gekennzeichnet, dass**
die Fasern (1) eine Länge von 30-60 mm aufweisen, und jede der Komponenten (2, 3) in den Zweikomponentenfasem (1)
aus einer unterschiedlichen Art von Polypropylen besteht oder die Komponenten (2, 3) der Zweikomponentenfasern (1) Polypropylen bzw. Polyester sind.

2. Oberflächenmaterial nach Anspruch 1,
**dadurch gekennzeichnet, dass**
die Fasern eine Länge von 40-50 mm aufweisen.

3. Oberflächenmaterial nach Anspruch 1 oder 2,
**dadurch gekennzeichnet, dass**
das Material eine Mischung von Zweikomponentenfasern aufweist.

4. Oberflächenmaterial nach einem der vorangehenden Ansprüche,
**dadurch gekennzeichnet, dass**
das Material ferner thermoplastische Bindefasern aus beispielsweise Polypropylen oder Polyethylen auf weist.

5. Oberflächenmaterial nach einem der vorangehenden Ansprüche,
**dadurch gekennzeichnet, dass**
die Zweikomponentenfasern eine Dicke von etwa 1,5-' 3,3 dtex oder 1,7-2,5 dtex aufweisen.

6. Oberflächenmaterial nach einem der vorangehenden Ansprüche,
**dadurch gekennzeichnet, dass**
das Material ein Quadratmetergewicht von etwa 40-100 g/m² oder 50-90 g/m² aufweist.

7. Absorptionsartikel, wie z.B. Windel, Hygienebinde, Inkontinenzschutz, Slipeinlage oder ähnlicher Artikel, mit einer flüssigkeitsundurchlässigen Rücklage (6), einer Absorptionslage oder -schicht (7) und einer flüssigkeitsdurchlässigen Oberflächenlage (5), die dafür vorgesehen ist, bei der Verwendung proximal zu dem Benutzer zu liegen, wobei das Oberflächenlagenmaterial wärmegeschrumpfte, spiralisierte, elastische, thermoplastische Zweikomponentenfasern (1) aufweist,
wobei die Komponenten (2, 3) in den Fasern zueinander unterschiedliche Schrumpfungseigenschaften aufweisen und in einer Seite-an-Seite-Beziehung liegen, und wobei die Komponenten (2, 3) wasserabweisend sind,
**dadurch gekennzeichnet, dass**
die Fasern (1) eine Länge von 30-60 min aufweisen, und die Komponenten (2, 3) in den Zweikomponentenfasern (1) aus zueinander unterschiedlichen Arten von Polypropylen bestehen, oder die Komponenten (2, 3) in den Zweikomponentenfasern Polypropylen bzw. Polyester sind.

8. Absorptionsartikel nach Anspruch 7,
**dadurch gekennzeichnet, dass**
die Fasern (1) eine Länge von 40-50 mm aufweisen.

9. Absorptionsartikel nach Anspruch 7 oder 8,
**dadurch gekennzeichnet, dass**
die Oberflächenlage (5) eine kombinierte Oberflächenlage und Erfassungs-/Transportlage bildet.

10. Absorptionsartikel nach Anspruch 7 oder 8,
**dadurch gekennzeichnet, dass**
das Oberflächenlagenmaterial eine Mischung von Zweikomponentenfasern aufweist.

11. Absorptionsartikel nach einem der Ansprüche 7 bis 10,
**dadurch gekennzeichnet, dass**
das Oberflächenmaterial (5) ferner thermoplastische Bindefasern aus beispielsweise Polypropylen oder Polyethylen aufweist.

12. Absorptionsartikel nach einem der vorangehenden Ansprüche,
**dadurch gekennzeichnet, dass**
die Zweikomponentenfasern eine Dicke von etwa 1,5-3,3 dtex oder 1,7-2,5 dtex aufweisen.

13. Absorptionsartikel nach einem der vorangehenden Ansprüche,
**dadurch gekennzeichnet, dass**
die Oberflächenlage ein Quadratmetergewicht von etwa 40-100 g/m² oder 50-90 g/m² aufweist.

14. Verwendung eines Materials, das wärmegeschrumpfte, spiralisierte, elastische, thermoplastische Zweikomponentenfasern (1) aufweist, wobei die Komponenten (2, 3) der Fasern wasserabweisend sind und zueinander unterschiedliche Schrumpfungseigenschaften aufweisen und in einer Seite-an-Seite-Beziehung liegen, als eine kombinierte Oberflächenlage und Erfassungs/Transportlage (5) in Absorptionsartikeln, wie z.B. Windeln, Hygienebinden, Inkontinenzschutzeinrichtungen, Slipeinlagen und ähnlichen Artikeln, die unterhalb der kombinierten Oberflächenlage und Erfassungs/Transportlage (5) eine Absorptionslage (7) und eine flüssigkeitsundurchlässige Rücklage (6) aufweisen,
**dadurch gekennzeichnet, dass**
die Fasern eine Länge von 30-60 mm aufweisen, und die Komponenten (2, 3) der Zweikomponentenfasern (1) zueinander unterschiedliche Arten von Polypropylen sind, oder die Komponenten (2, 3) der Zweikomponentenfasern (1) Polypropylen bzw. Polyester sind.

15. Verwendung eines Materials nach Anspruch 14, wobei die Fasern (1) eine Länge von 40-50 mm aufweisen.

16. Verwendung eines Materials nach Anspruch 14 oder 15,
wobei das Material eine Mischung von Zweikomponentenfasern aufweist.

17. Verwendung eines Materials nach einem der Ansprüche 14 bis 16,
wobei
das Material ferner thermoplastische Bindefasern aus beispielsweise Polypropylen oder Polyethylen aufweist.

18. Verwendung eines Materials nach einem der vorangehenden Ansprüche,
wobei
die Zweikomponentenfasern eine Dicke von etwa 1,5-3,3 dtex oder 1,7-2,5 dtex aufweisen.

19. Verwendung eines Materials nach einem der vorangehenden Ansprüche,
wobei
das Material ein Quadratmetergewicht von etwa 40-100 g/m² oder 50-90 g/m² aufweist.

## Revendications

1. Matériau superficiel pour des articles absorbants tels que des couches-culottes, des serviettes hygiéniques, des protections contre l'incontinence, des protège-slips, et autres articles similaires, lequel matériau superficiel (5) comprenant des fibres à deux composants, thermoplastiques, élastiques, en spirale, et thermorétractées (1), les composants (2, 3) dans lesdites fibres présentant des propriétés de retrait différentes de l'un à l'autre, et s'étendant côte-à-côte, et les composants (2, 3) étant hydrophobes, **caractérisé en ce que** les fibres (1) présentent une longueur de 30-60 mm, et les composants (2, 3) dans les fibres à deux composants (1) étant constitués chacun d'un type différent de polypropylène ou les composants (2, 3) des fibres à deux composants (1) sont respectivement en polypropylène et en polyester.

2. Matériau superficiel selon la revendication 1, **caractérisé en ce que** les fibres présentent une longueur de 40-50 mm.

3. Matériau superficiel selon la revendication 1 ou 2, **caractérisé en ce qu'**il comprend un mélange de fibres à deux composants.

4. Matériau superficiel selon l'une quelconque des rerendications précédentes, **caractérisé en ce qu'**il comprend également des fibres liantes thermoplastiques de polypropylène ou de polyéthylène, par exemple.

5. Matériau superficiel selon l'une quelconque des revendications précédentes, **caractérisé en ce que** les fibres à deux composants présentent une épaisseur d'environ 1,5-3,3 dtex ou 1,7-2,5 dtex.

6. Matériau superficiel selon l'une quelconque des revendications précédentes, **caractérisé en ce qu'**il présente un poids par unité de surface d'environ 40-100 g/m² ou 50-90 g/m².

7. Article absorbant, tel qu'une couche-culotte, une serviette hygiénique, une protection contre l'incontinence, un protège-slip, ou autre article similaire, comprenant une feuille de renforcement imperméable au liquide (6), une couche ou feuille absorbante (7), et une feuille superficielle supérieure perméable au liquide (5) qui est destinée à se trouver à proximité de l'utilisateur pendant l'utilisation, dans lequel le matériau en feuille superficiel comprend des fibres à deux composants, thermoplastiques, élastiques, en spirale, et thermorétractées (1), les composants (2, 3) dans lesdites fibres présentant des propriétés de retrait différentes de l'un à l'autre et s'étendant côte-à-côte, et les composants (2, 3) étant hydrophobes, **caractérisé en ce que** les fibres (1) présentent une longueur de 30-60 mm, et **en ce que** les composants (2, 3) dans les fibres à deux composants (1) sont constitués chacun d'un type différent de polypropylène ou les composants (2, 3) des fibres à deux composants (1) sont respectivement en polypropylène et en polyester.

8. Article absorbant selon la revendication 7, **caractérisé en ce que** les fibres présentent une longueur de 40-50 mm.

9. Article absorbant selon la revendication 7 ou 8, **caractérisé en ce que** la feuille superficielle (5) forme une feuille superficielle et une feuille d'acquisition/transport combinées.

10. Article absorbant selon la revendication 7 ou 8, **caractérisé en ce que** le matériau en feuille superficiel comprend un mélange de fibres à deux composants.

11. Article absorbant selon l'une quelconque des revendications 7 à 10, **caractérisé en ce que** le matériau superficiel (5) comprend également des fibres liantes thermoplastiques de polypropylène ou de polyéthylène, par exemple.

12. Article absorbant selon l'une quelconque des revendications précédentes, **caractérisé en ce que** les fibres à deux composants présentent une épaisseur d'environ 1,5-3,3 dtex ou 1,7-2,5 dtex.

13. Article absorbant selon l'une quelconque des revendications précédentes, **caractérisé en ce que** la feuille superficielle présente un poids par unité de surface d'environ 40-100 g/m² ou 50-90 g/m².

14. Utilisation d'un matériau qui comprend des fibres à deux composants, thermoplastiques, élastiques, en spirale, et thermorétractées (1), les composants (2, 3) desdites fibres étant hydrophobes et présentant des propriétés de retrait différentes de l'un à l'autre et s'étendant côte-à-côte, comme feuille superficielle et feuille d'acquisition/transport (5) combinées dans des articles absorbants tels que des couches-culottes, des serviettes hygiéniques, des protections contre l'incontinence, des protège-slips, et autres articles similaires, qui comprennent en dessous de la feuille superficielle et de la feuille d'acquisition/transport (5) combinées une feuille absorbante (7) et une feuille de renforcement imperméable au liquide (6), **caractérisée en ce que** les fibres présentent une longueur de 30-60 mm, et les composants (2, 3) des fibres à deux composants (1) sont des polypropylènes différents l'un de l'autre ou les composants (2, 3) des fibres à deux composants (1) sont respectivement en poplypropylène et en polyester.

15. Utilisation d'un matériau selon la revendication 14, dans laquelle les fibres (1) présentent une longueur de 40-50 mm.

16. Utilisation d'un matériau selon la revendication 14 ou 15, dans laquelle le matériau comprend un mélange de fibres à deux composants.

17. Utilisation d'un matériau selon l'une quelconque des revendications 14 à 16, dans laquelle le matériau comprend en outre des fibres liantes thermoplastiques de polypropylène ou de polyéthylène, par exemple.

18. Utilisation d'un matériau selon l'une quelconque des revendications précédentes, dans laquelle les fibres à deux composants présentent une épaisseur d'environ 1,5-3,3 dtex ou 1,7-2,5 dtex.

19. Utilisation d'un matériau selon l'une quelconque des revendications précédentes, dans laquelle le matériau présente un poids par unité de surface d'environ 40-100 g/m² ou 50-90 g/m².
